# EUROPEAN PATENT SPECIFICATION

(11) **EP 1 610 675 B1**
(45) Date of publication and mention of the grant of the patent: **19.08.2009**
(21) Application number: 03816574.2
(22) Date of filing: 08.04.2003
(51) Int. Cl.: A61B 5/0215, A61B 5/03, G01L 9/18

(54) **APPARATUS FOR MEASURING PRESSURES DERIVING FROM LEAKAGE CURRENT**
VORRICHTUNG ZUM MESSEN VON AUS LECKSTROM STAMMENDEM DRUCK
APPAREIL DE MESURE DE PRESSIONS SUR LA BASE DE COURANTS DE FUITE

(43) Date of publication of application: 04.01.2006
(73) Proprietor: PP-Technologies AG, 3186 Düdingen (CH)
(72) Inventor: GLOCKER, Raymond, c/o PP-Technologies AG, 3186 Düdingen (CH); ÖZDEMIR, Haldun, c/o PP-Technologies AG, 3186 Düdingen (CH)
(74) Representative: Besse, François
(86) International application number: PCT/CH2003/000227
(87) International publication number: WO 2004/089207

(56) References cited:
- WO-A-84/00290
- US-B1- 6 450 972
- KNOLL M ET AL: "Pressure profile sensing system" , SENSORS AND ACTUATORS A, ELSEVIER SEQUOIA S.A., LAUSANNE, CH, VOL. 93, NR. 1, PAGE(S) 52-56 XP004255506 ISSN: 0924-4247 cited in the application the whole document

## Description

### Field of the Invention

The invention refers to medical investigation or diagnostic tools and methods applicable to mammals, more specifically to methods for measuring pressures, respectively pressure profiles in various body tracts or cavities.

### Background of the invention

Among various technologies available for measuring pressures within the frame of medical investigations or diagnostic methods the Pressure Profile Sensor Method has been developed recently (PPS-Method). US Patent 6,450,972 of September 17, 2002 and the literature cited therein provide detailed information concerning the fundamentals of that technique and its potential uses - see also "Pressure Profile Sensing Systems" in Sensors and Actuators A 93 (2001), 52-56, Elsevier Science B.V. Document US-B-6 450 972 represents the most relevant prior art.

The basis of that highly performing technique is linked to the deformation of the shape of a saline solution caused by an external pressure when applied to a catheter lumen in which the saline solution is located provided the walls of the catheter tubing in which the saline solution is filled in are thin enough to be deflected by the external pressure. As the saline solution is electrically conducting, the capacitance of the same will change when the shape is changed through the applied pressure along the axis of the tubing and over time, the most sensitive part of the saline solution column being the front of same. So variations of the pressure can be followed over time by appropriate capacitive measurements.

Capacitance measurements as referred to here above, however, necessitate the use of expensive and complex material especially that used for manufacturing catheters incorporating conductive means. Accuracy and sensitivity of current methods also need improvement.

Consequently the medical community is still looking for easier, more efficient, more precise and more reliable methods and, if ever possible, less painful for the patients. The present invention provides a very efficient solution which easily overcomes all the obstacles currently met.

### Description of the invention

The invention is defined in claim 1.

Specific embodiments of the apparatus defined in claim 1 shall appear when relevant, in the description here below.

The alternative current which applies to the liquid substance is in fact a low voltage/high frequency current. "Low voltage" means a current voltage which is easily supported by the patient, namely not detrimental to sensitive body parts or organs, and not painful, but strong enough to provide electrical excitations which will be eventually detected by means such an external electrode. Typical voltage which applies according to the invention is comprised between about 500 mV and about 6 V, preferably between about 1 V and about 4 V.

The frequency of the low voltage current applied to the liquid substance has to be high enough to go above the insulation barrier provided by the wall of the catheter currently used in that domain, more specifically of the magnitude of about 60 kHz. The upper value of said frequency will be defined as not interfering with e.g. natural or artificial heart stimulation and also not causing any pain to the patient. Said upper value, moreover, must also be fully compatible with the machines used according to the PPS-Method. Typically, said frequency will be comprised between about 60 kHz and about 130 kHz, preferably between about 80 kHz and about 120 kHz.

Preferred conditions for such an alternative current are 2 V / 100 kHz; they fulfill all the regulatory and technical requirements currently met worldwide. The source of such an alternative current will be selected from tools usually met in the prior known technology.

The basic concept of the present invention resides in the fact that the electrically excited liquid column is subjected to mechanical oscillations. Said mechanical oscillations have controlled amplitude and frequency, the amplitude being of about max. 4 mm, preferably of about 2 mm and the frequency of about max 15, preferably of about 10 Hz. These values have proved non-disturbing the homogeneity of the liquid column currently used for such measurements, and consequently compatible with the accuracy of pressure measurements which is required. Related detailed explanations can be found in e.g. EP 02 405 490.0 filed June 14, 2002 published as EP-A-1371325.

According to the invention the electrically conductive liquid substance is moving progressively, either continuously or stepwise, through the catheter. Continuous liquid introduction can occur for moving the frontline of the liquid column to a certain location whereas step-by-step progression occurs when measurements take place. Said progression is achieved by means of a peristaltic pump directly fitted to the liquid source - see also EP 02 405 490.0 -.

The mechanical oscillation means are located downwards to the peristaltic pump and comprise e.g. a stepper motor which can be triggered fast or slow to generate high or low frequency oscillations while controlling simultaneously the amplitude of the oscillations - see also EP 02 405 490.0 -.

According to the invention the catheter has a portion which is sufficiently flexible to be deflected by the pressure which will be exerted on it once introduced into the subject. Said portion is preferably a longitudinal strip of the catheter which is thinner than the remaining portion of the catheter wall. Such a construction is easily achievable using the polymer extrusion or co-extrusion techniques.

Whenever required the catheter can be conveniently reinforced internally by a suitable fiber web.

The catheter is most frequently a single lumen catheter. It can also be a multi-lumen catheter as that described in PCT/CH 03/0113 filed February 17, 2003 published as WO-A-2004/071293.

Said catheter is made of innocuous polymer plastic material, preferably of non-conductive polymer material and it does not need to comprise conductive material such as metal strips or inner metal coating. This allows manufacturing thin and flexible tubes which are cheaper and easier to handle than those used for the prior known technology - see e.g. US 6,459,972. Such catheters revealed furthermore definitely less painful for the patients. Suitable polymer material can be selected among silicon, rubber, latex, polyurethane, PVC, polypropylene, PE or the like.

According to the invention the electrically conductive liquid substance is an aqueous liquid like a saline solution, e.g. a NaCl or KCI solution.

According to the invention the leakage current induced by the electrically excited saline solution progressing through the catheter is recorded by a currently available electrode placed at the external surface of the subject body. One of the main features of the present invention resides in the fact that the leakage current thus induced is strong and homogenous enough to reflect accurately the pressure values measured at a given location. When compared to prior known PPS-Method e.g. according to US 6,459,972, which is based on capacitance measurements, the method according to the present invention proved definitely more sensitive and more precise for the same electrical excitation parameters.

Convenient recording of the leakage current is performed by means of one external electrode only, which can be fixed at any suitable body part. The leakage current is then transferred to a converter suitable to convert leakage current parameters into corresponding pressure values which can be displayed eventually in the form of graphs e.g. on paper strips or computer screens.

Pressure values may appear as such for a given location and at a given time. They can also be calculated for a given location but over a certain period and so display the pressure variation or evolution over time; they can be furthermore detected while the front line of the electrically excited liquid is progressing step-by-step through the catheter to afford corresponding pressure profiles. Further details of these types of measurements appear in EP 02 405 490.0.

According to the invention, eventually, the method can be used to for perform real time pressure, respectively pressure profile measurements. It can be used also to perform ex-temporaneum pressure, respectively pressure profiles measurements as well, by recording the pressure values provided by the converter and by displaying them at a time different from that of the leakage current recording.

The method described here above has proved highly efficient for performing pressure, respectively pressure profile measurements in mammal body tracts or cavities such as lung, esophagus, stomach, intestine, urinary tract or bladder, or blood vessels.

## Claims

1. An apparatus for performing pressure, respectively pressure profile, measurements in mammals by means of the pressure profile sensors technique, which comprises :
- an alternative current source;
- a catheter having at least a portion of its wall which is sufficiently flexible to be deflected by external pressure
- a source of an electrically conductive liquid substance connected to said alternative current source;
- peristaltic pumping means fitted directly to the source of liquid substance;
- mechanical oscillation means connected downwards to peristaltic pumping means;
- an electrode adapted to be placed at the external surface of the subject for recording and then transferring a detected leakage current to the converter; the leakage current being induced by the liquid substance travelling through the catheter due to said electrically conductive liquid substance being introduced progressively into the catheter lumen while applying simultaneously to said electrically conductive liquid substance alternative current from said current source and mechanical oscillations from said mechanical oscillation means;
- a converter suitable for deriving pressure values from the leakage current parameters which have been transferred thereto; and
- means suitable to display pressure values as such, or as a function of the measurement location or measurement period or both.

2. Apparatus according to claim 1, wherein the alternative current source is a low voltage/high frequency current source and wherein the mechanical oscillations means are adapted to control amplitude and frequency of the mechanical oscillations.

3. Apparatus according to claim 1 or 2 comprising a catheter, wherein said catheter is made of innocuous polymer plastic material, preferably of non-conductive innocuous polymer plastic material.

4. Apparatus according to claim 3, wherein the catheter is a single lumen or a multi-lumen catheter.

5. Apparatus according to any of claim 1 to 4, wherein the electrically conductive liquid substance is an aqueous liquid, preferably a saline solution.

## Patentansprüche

1. Vorrichtung zum Durchführen von Druck- bzw. Druckprofilmessungen in Säugetieren mit Hilfe der Druckprofilsensorentechnik, die folgendes umfaßt:
- eine Wechselstromquelle;
- einen Katheter mit mindestens einem Abschnitt von seiner Wand, der ausreichend flexibel ist, um durch äußeren Druck ausgelenkt zu werden;
- eine Quelle für eine elektrisch leitende flüssige Substanz, an die Wechselstromquelle angeschlossen;
- direkt an die Quelle für flüssige Substanz angebrachte Peristaltikpumpmittel;
- abwärts zu Peristaltikpumpmitteln angeschlossene mechanische Oszillationsmittel;
- eine Elektrode, die dafür ausgelegt ist, an der äußeren Oberfläche des Subjekts plaziert zu werden zum Aufzeichnen und dann Transferieren eines detektierten Leckstroms zu dem Konverter; wobei der Leckstrom durch die flüssige Substanz induziert wird, die sich durch den Katheter aufgrund der elektrisch leitenden flüssigen Substanz bewegt, die progressiv in das Katheterlumen eingeleitet wird, während simultan an die elektrisch leitende flüssige Substanz Wechselstrom von der Stromquelle und mechanischen Oszillationen von dem mechanischen Oszillationsmittel angelegt werden;
- einen Konverter, der sich dafür eignet, Druckwerte aus Leckstromparametern, die dorthin transferiert worden sind, abzuleiten; und
- Mittel, die sich eignen, um Druckwerte als solche oder als Funktion des Meßorts oder der Meßperiode oder beider anzuzeigen.

2. Vorrichtung nach Anspruch 1, wobei die Wechselstromquelle eine Niederspannungs-/Hochfrequenzstromquelle ist und wobei die mechanischen Oszillationsmittel dafür ausgelegt sind, Amplitude und Frequenz der mechanischen Oszillationen zu steuern.

3. Vorrichtung nach Anspruch 1 oder 2, umfassend einen Katheter, wobei der Katheter aus harmlosem Polymerkunststoffmaterial hergestellt ist, bevorzugt aus nichtleitendem harmlosem Polymerkunststoffmaterial.

4. Vorrichtung nach Anspruch 3, wobei der Katheter ein Katheter mit einzelnem Lumen oder mehreren Lumen ist.

5. Vorrichtung nach einem der Ansprüche 1 bis 4, wobei die elektrisch leitende flüssige Substanz eine wäßrige Flüssigkeit ist, bevorzugt eine Salzlösung.

## Revendications

1. Appareil destiné à réaliser des mesures de pression, respectivement de profil de pression, chez des mammifères au moyen de la technique avec capteurs de profil de pression, comprenant :
- une source de courant alternatif ;
- un cathéter dont au moins une partie de la paroi est suffisamment souple pour être déformée par une pression externe ;
- une source de substance liquide conductrice de l'électricité reliée à ladite source de courant alternatif ;
- un moyen de pompage péristaltique monté directement sur la source de substance liquide ;
- un moyen d'oscillations mécaniques relié vers le bas au moyen de pompage péristaltique ;
- une électrode prévue pour être placée au niveau de la surface externe du sujet en vue d'enregistrer puis de transmettre un courant de fuite détecté au convertisseur ; le courant de fuite étant induit par la circulation de la substance liquide à travers le cathéter du fait de l'introduction progressive de ladite substance liquide conductrice de l'électricité dans la lumière du cathéter tandis qu'on applique simultanément à ladite substance liquide conductrice de l'électricité un courant alternatif provenant de ladite source de courant et des oscillations mécaniques provenant dudit moyen d'oscillations mécaniques ;
- un convertisseur approprié pour déterminer des valeurs de pression à partir des paramètres de courant de fuite qui lui ont été transmis ; et
- un moyen approprié pour afficher des valeurs de pression en tant que telles, ou en fonction de l'emplacement et / ou de la période de mesure.

2. Appareil selon la revendication 1, la source de courant alternatif étant une source de courant à basse tension / haute fréquence et le moyen d'oscillations mécaniques étant prévu pour commander l'amplitude et la fréquence des oscillations mécaniques.

3. Appareil selon la revendication 1 ou 2, comportant un cathéter, ledit cathéter étant constitué d'un matériau polymère plastique inoffensif, de préférence d'un matériau polymère plastique inoffensif non conducteur.

4. Appareil selon la revendication 3, le cathéter étant un cathéter à lumière simple ou un cathéter à lumières multiples.

5. Appareil selon l'une quelconque des revendications 1 à 4, la substance liquide conductrice de l'électricité étant un liquide aqueux, de préférence une solution saline.
